(19) Europäisches Patentamt
European Patent Office
Office européen des brevets

(11) Veröffentlichungsnummer : **0 071 153**
**B1**

(12) # EUROPÄISCHE PATENTSCHRIFT

(45) Veröffentlichungstag der Patentschrift : **10.09.86**

(21) Anmeldenummer : **82106524.0**

(22) Anmeldetag : **20.07.82**

(51) Int. Cl.[4] : **C 07 J 1/00**, C 07 J 53/00, A 61 K 31/565// C07J21/00, C07J71/00

(54) **Neue Androstan-Derivate, Verfahren zu ihrer Herstellung und pharmazeutische Präparate, die diese Verbindungen enthalten.**

(30) Priorität : **29.07.81 DE 3130644**

(43) Veröffentlichungstag der Anmeldung : **09.02.83 Patentblatt 83/06**

(45) Bekanntmachung des Hinweises auf die Patenterteilung : **10.09.86 Patentblatt 86/37**

(84) Benannte Vertragsstaaten : **AT BE CH DE FR GB IT LI LU NL SE**

(56) Entgegenhaltungen :
**FR-A- 2 342 990**
**FR-A- 2 391 224**
**FR-M- 7 871**
**Die Akte enthält technische Angaben, die nach dem Eingang der Anmeldung eingereicht wurden und die nicht in dieser Patentschrift enthalten sind.**

(73) Patentinhaber : **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder : **Bittler, Dieter**
**Bölkauer Pfad**
**D-1000 Berlin 27 (DE)**
Erfinder : **Laurent, Henry, Dr.**
**Glambecker Weg 21**
**D-1000 Berlin 28 (DE)**
Erfinder : **Nickisch, Klaus, Dr.**
**Alt-Lichtenrade 11**
**D-1000 Berlin 49 (DE)**
Erfinder : **Wiechert, Rudolf, Dr. Prof.**
**Petzower Strasse 8a**
**D-1000 Berlin 39 (DE)**
Erfinder : **Albring, Manfred, Dr.**
**Im Ellenbügel 81**
**D-6456 Langenselbold (DE)**
Erfinder : **Schleusener, Annerose, Dr.**
**Ilsensteinweg 1 B**
**D-1000 Berlin 38 (DE)**

**EP 0 071 153 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft
Androstan-Derivate der allgemeinen Formel I

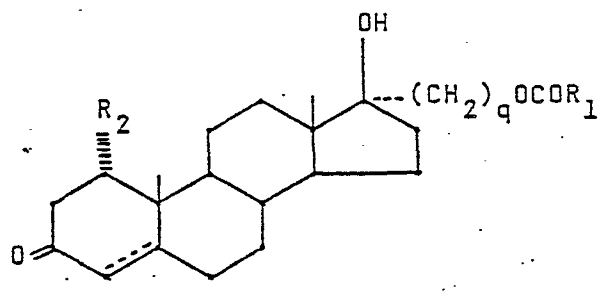

(I)

worin ..... eine Einfachbindung oder eine Doppelbindung $R_1$ einen Alkylrest mit 1 bis 8 Kohlenstoffatomen, q die Ziffern 3 oder 4 und $R_2$ ein Wasserstoffatom oder eine Methylgruppe darstellen.

Die erfindungsgemäßen Androstan-Derivate der allgemeinen Formel I können als Substituenten $R_2$ einen geradkettigen oder verzweigten Alkylrest mit 1 bis 8 Kohlenstoffatomen tragen. Geeignete Alkylreste R sind beispielsweise der Methylrest, der Äthylrest, der Propylrest, der Isopropylrest, der Butylrest, der Isobutylrest, der tert.-Butylrest, der Pentylrest, der Hexylrest oder der Octylrest.

Die Androstan-Derivate der allgmeinen Formel I zeichnen sich bei lokaler Applikation überraschender Weise durch eine ausgeprägte sebumsuppressive Wirkung aus. Bei systemischer Applikation zeigen diese Verbindungen keine endokrinen Nebenwirkungen. In allen Testen auf östrogene, antiöstrogene, androgene, antiandrogene und gestagene Wirkung waren sie unwirksam.

Demgegenüber sind die in den französischen Patentschriften 787 111, 2 391 224 und 2 342 990 beschriebenen Verbindungen bei topischer Applikation nicht oder nur gering sebumsupressiv wirksam.

Die sebumsuppressive Wirkung wurde durch Untersuchungen zur Beeinflussung der Talgdrüsen des Hamsters bestimmt. Die Verbindungen der allgemeinen Formel I hemmen die Lipidsynthese in den Talgdrüsen der Hamsterohren und verkleinern die Areale der Talgdrüsen und die Flankenorgane.

Zur topischen Anwendung können die erfindungsgemäßen Verbindungen mit den üblichen Trägersubstanzen zu Lösungen, Gels, Salben, Puder oder anderen Präparaten verarbeitet werden. Geeignete Trägersubstanzen sind zum Beispiel Wasser, Ethanol, Propanol, Glycerin, Methylcellulose, Hydroxypropyl-cellulose, Carboxypolymethylen usw.. Das Sebumsuppressivum liegt vorzugsweise in einer Konzentration von 0,05 bis 5,0 Gewichts-%, bezogen auf das Gesamtgewicht des Präparates, vor. Die Präparate können zur topischen Behandlung von Erkrankungen wie Akne und Seborrhoe verwendet werden.

Die neuen Androstan-Derivate können nach einem Verfahren hergestellt werden, das unter Bedingungen durchgeführt werden kann, die dem Fachmann wohlbekannt sind, und das dadurch gekennzeichnet ist, daß man in an sich bekannter Weise

a) ein Androstan-Derivat der allgemeinen Formel II

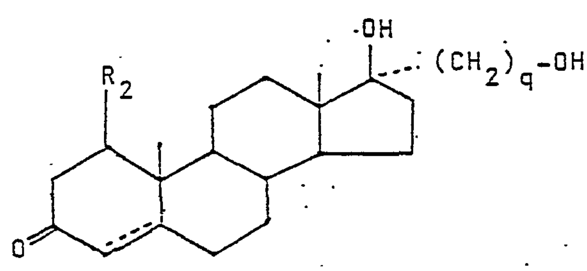

(II)

worin ....., $R_2$ und q die obengenannte Bedeutung besitzen, mit einer Säure der allgemeinen Formel III

$R_1COOH$ (III),

worin $R_1$ die obengenannte Bedeutung besitzt, oder einem reaktionsfähigen Derivat derselben, verestert oder

b) aus einem Androstan-Derivat der allgemeinen Formel IV

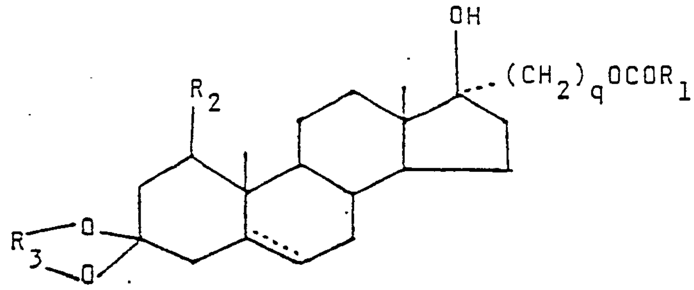

(IV)

worin ....., $R_1$, $R_2$ und q die obengenannte Bedeutung besitzen, und $R_3$ eine geradkettige oder verzweigte Alkylengruppe mit 2 bis 6 Kohlenstoffatomen darstellt, die Ketalgruppe hydrolytisch abspaltet, oder

c) ein Androstan-Derivat der allgemeinen Formel V

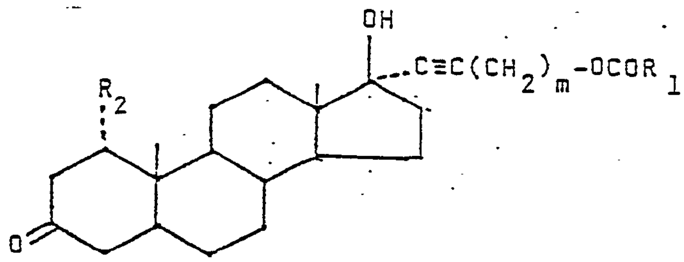

(V)

worin $R_1$ und $R_2$ die obengenannte Bedeutung besitzt und m die Ziffer q — 2 darstellt, zur Herstellung gesättigter 1α,2α-Methylensteroide der allgemeinen Formel I hydriert.

So kann man beispielsweise das Verfahren in der Weise durchführen, daß man die Androstan-Derivate der allgemeinen Formel II mit den Säurechloriden oder Säureanhydriden in Gegenwart von Basen wie Kaliumkarbonat, Pyridin oder Collidin verestert. Andererseits ist es aber auch möglich, diese Verbindungen mit den freien Säuren in Gegenwart von Veresterungskatalysatoren wie Carbonyldiimidazol, Dicyclohexylcarbodiimid oder Trifluoressigsäureanhydrid zu verestern.

Zur Durchführung der Verfahrensvariante b kann man beispielsweise die Androstan-Derivate der allgemeinen Formel III mit einer wasserhaltigen Carbonsäure — wie zum Beispiel Essigsäure — oder einer wässrigen Mineralsäure in Gegenwart eines geeigneten Lösungsmittels wie Dioxan, Tetrahydrofuran oder Glycoldimethyläther hydrolysieren.

Zur Durchführung der Verfahrensvariante c werden die Androstan-Derivate der allgemeinen Formel V beispielsweise in einem inerten Lösungsmittel wie Äthanol, Isopropanol, Ethylacetat, Tetrahydrofuran, Dioxan, Benzol, Toluol etc. in Gegenwart eines Platin- oder Palladiumkatalysators wie Platinoxidkatalysatoren, Palladium-Tierkohlekatalysatoren oder Lindlar-Katalysator (L.F. Fieser und M Fieser, Reagents for Organic Synthesis ; John Wiley & Sons. Inc., New York etc. 1967, 566) hydriert.

Die Ausgangsverbindungen für das erfindungsgemäße Verfahren sind bekannt, oder können in an sich bekannter Weise hergestellt werden, wie dies in den nachfolgenden Ausführungsbeispielen erläutert ist.

## Beispiel 1

A. Eine Lösung von 30 g 17β-Hydroxy-1α-methyl-4-androsten-3-on in 1250 ml Benzol versetzt man mit 340 ml Ethylenglykol und 1.7 g p-Toluolsulfonsäure und destilliert das sich bei der Reaktion bildende Wasser innerhalb von 15 Stunden azeotrop ab. Nach dem Abkühlen der Reaktionsmischung wird diese mit 50 ml Pyridin versetzt, mit Wasser gewaschen, über Natriumsulfat getrocknet und im Vakuum eingeengt. Der Rückstand wird an Kieselgel chromatographiert. Mittels Hexan-Ethylacetat-Gradienten (43-65 % Ethylacetat) eluiert man 20.7 g 3,3-Ethylendioxy-1α-methyl-5-androsten-17β-ol.

B. 20.7 g 3,3-Ethylendioxy-1α-methyl-5-androsten-17β-ol werden in 470 ml Dichlormethan gelöst. Die Lösung wird mit 20.7 g Pyridiniumdichromat versetzt, 15 Stunden bei Raumtemperatur gerührt, anschließend mit Wasser gewaschen, getrocknet und im Vakuum eingedampft. Der Rückstand wird an Kieselgel chromatographiert und man eluiert mittels Hexan-Ethylacetat-Gradienten (16-25 % Ethylacetat) 14.2 g 3,3-Ethylendioxy-1α-methyl-5-androsten-17-on.

C. Zu einer Lösung von 7.6 g 3,3-Ethylendioxy-1α-methyl-5-androsten-17-on in 76 ml wasserfreiem Tetrahydrofuran tropft man in einer Argonatmosphäre, nach Zugabe von 12.6 g Kaliumethylat, 7.6 ml Propargylalkohol gelöst in 7.6 ml Tetrahydrofuran innerhalb von 25 Minuten. Das Reaktionsgemisch wird danach noch 3 Stunden bei Raumtemperatur gerührt, unter Eiskühlung mit 9.8 ml konzentrierter Essigsäure versetzt und im Vakuum weitgehend eingeengt. Der Rückstand wird mit Wasser versetzt und

mit Ethylacetat extrahiert, der Extrakt wird mit Wasser gewaschen, getrocknet und im Vakuum eingedampft. Das Rohprodukt wird mit einem Aceton-Dichlormethan-Gradienten an Kieselgel chromatographiert. Mit 21-28 % Aceton werden 6.0 g 3,3-Ethylendioxy-17α-(3-hydroxy-1-propinyl)-1α-methyl-5-androsten-17β-ol eluiert. Eine Probe, die aus Aceton-Diisopropylether umkristallisiert wurde, schmilzt bei 207 °C. $[\alpha]_D^{25} = -84°$ (C = 0.5 in Chloroform).

D. 19.5 g 3,3-Ethylendioxy-17α-(3-hydroxy-1-propinyl)-1α-methyl-5-androsten-17β-ol werden in einer Mischung aus 100 ml Tetrahydrofuran und 140 ml Isopropylalkohol gelöst und nach Zugabe von 900 mg Palladium-Calciumcarbonat-Katalysator hydriert. Nach 100 Minuten beträgt die Wasserstoffaufnahme 2 225 ml bei 1 018 mb und 21 °C. Der Katalysator wird über eine Glasfilternutsche abgesaugt, das Filtrat im Vakuum zur Trockne eingedampft und der Rückstand an Kieselgel mit einem Aceton-Dichlormethan-Gradienten chromatographiert. Mit 32-50 % Aceton erhält man, nach dem Umkristallisieren aus Aceton-Diisopropylether, 10.15 g 3,3-Ethylendioxy-17α-(3-hydroxypropyl)-1α-methyl-5-androsten-17β-ol mit einem Schmelzpunkt von 161 °C. $[\alpha]_D^{25} = -36°$ (C = 0.5 in Chloroform).

E. 3.0 g 3,3-Ethylendioxy-17α-(3-hydroxypropyl)-1α-methyl-5-androsten-17β-ol werden mit 30 ml Pyridin und 15 ml Acetanhydrid versetzt. Das Gemisch läßt man 15 Stunden bei Raumtemperatur stehen und isoliert anschließend das Reaktionsprodukt durch Einrühren in Eiswasser und Extraktion mit Dichlormethan. Die Dichlormethanlösung wird getrocknet, in Vakuum eingedampft und der erhaltene Rückstand in 100 ml 90 %iger Essigsäure gelöst. Die Lösung wird 30 Minuten auf dem Dampfbad erhitzt und danach in Vakuum bei erhöhter Temperatur bis zur Trockne eingedampft. Der Rückstand wird an Kieselgel mit einem Hexan-Aceton-Gradienten chromatographiert. Mit 33-39 % Aceton erhält man, nach dem Umkristallisieren aus Diethylether-Petroleumbenzin, 2.98 g 17α-(3-Acetoxypropyl)-17β-hydroxy-1α-methyl-4-androsten-3-on. Schmelzpunkt 71 °C. $[\alpha]_D^{25} = +70°$ (C = 0.5 in Chloroform). UV : $\varepsilon_{243} = 13\,400$ (in Methanol).

## Beispiel 2

A. Eine Lösung von 2.0 g 3,3-Ethylendioxy-17α-(3-hydroxypropyl)-1α-methyl-5-androsten-17β-ol in 60 ml 90 prozentiger Essigsäure wird zwei Stunden auf 60 °C erhitzt und anschließend im Vakuum bei 60 °C zur Trockne eingedampft. Der Rückstand wird mit einem Hexan-Aceton-Gradienten an Kieselgel chromatographiert. Mit 55-66 % Aceton erhält man, nach dem Umkristallisieren aus Aceton-Diisopropylether, 1.31 g 17β-Hydroxy-17α-(3-hydroxypropyl)-1α-methyl-4-androsten-3-on. Schmelzpunkt 195 °C. $[\alpha]_D^{25} = +91°$ (C = 0.5 in Chloroform). UV : $\varepsilon_{242} = 14\,800$ (in Methanol).

B. 1.0 g 17β-Hydroxy-17α-(3-hydroxypropyl)-1α-methyl-4-androsten-3-on löst man in einem Gemisch aus 10 ml Pyridin und 5 ml Propionsäureanhydrid und läßt die Lösung 15 Stunden bei Raumtemperatur stehen. Das Reaktionsprodukt wird durch Einrühren in Eiswasser gefällt, es wird abgesaugt, getrocknet und aus Diethylether-Pentan umkristallisiert. Die Ausbeute beträgt 1.07 g 17β-Hydroxy-1α-methyl-17α-(3-propionyloxypropyl)-4-androsten-3-on. Schmelzpunkt 105 °C. $[\alpha]_D^{25} = +73°$ (C = 0.5 in Chloroform). UV : $\varepsilon_{243} = 14\,500$ (in Methanol).

## Beispiel 3

500 mg 17β-Hydroxy-17α-(3-hydroxypropyl)-1α-methyl-4-androsten-3-on werden in 5 ml Pyridin und 2.5 ml Buttersäureanhydrid gelöst. Die Umsetzung erfolgt innerhalb von 15 Stunden bei 20 °C. Das Produkt wird durch Eingießen in Eiswasser gefällt. Nach einer Stunde wird abgesaugt, getrocknet und aus Diethylether-Hexan umkristallisiert. Man erhält 403 mg 17α-(3-Butyryloxypropyl)-17β-hydroxy-1α-methyl-4-androsten-3-on mit einem Schmelzpunkt von 94 °C. $[\alpha]_D^{25} = +71°$ (C = 0.5 in Chloroform). UV : $\varepsilon_{243} = 14\,900$ (in Methanol).

## Beispiel 4

A. Eine Lösung von 30 g 17β-Hydroxy-1α-methyl-5α-androstan-3-on in 300 ml Toluol und 90 ml Ethylenglykol wird mit 900 mg p-Toluolsulfonsäure versetzt und 6 Stunden unter langsamen Abdestillieren gerührt. Nach dem Abkühlen wird die Reaktionslösung mit 3 ml Pyridin versetzt, mit Diethylether verdünnt und mit Wasser gewaschen. Nach dem Eindampfen werden 35 g 3,3-Ethylendioxy-1α-methyl-5α-androstan-17β-ol erhalten.

B. 35 g 3,3-Ethylendioxy-1α-methyl-5α-androstan-17β-ol werden in 350 ml Dimethylformamid mit 70 g Pyridiniumdichromat 17 Stunden bei Raumtemperatur gerührt. Die Reaktionslösung wird in die 10fache Menge Ethylacetat eingerührt, danach wird von den ausgeschiedenen Chromsalzen abdekantiert und die organische Phase mit Wasser gewaschen. Nach dem Trocknen und Eindampfen werden 35 g 3,3-Ethylendioxy-1α-methyl-5α-androstan-17-on vom Schmelzpunkt 154 °C erhalten.

C. Eine Lösung von 10 g 3,3-Ethylendioxy-1α-methyl-5α-androstan-17-on in 100 ml wasserfreiem Tetrahydrofuran wird im Eisbad abgekühlt. Unter Argon werden 30 g Kaliumethylat eingetragen und anschließend 20 ml Propargylalkohol, gelöst in 40 ml Tetrahydrofuran, innerhalb 30 Minuten eingetropft. Die Reaktionslösung wird 5.5 Stunden bei Raumtemperatur nachgerührt, im Eisbad abgekühlt und mit 23 ml Essigsäure versetzt. Danach wird im Vakuum weitgehend eingeengt, der Rückstand in Ethylacetat

aufgenommen, die Lösung mit Wasser und gesättigter Natriumhydrogencarbonatlösung gewaschen, getrocknet und eingedampft. Aus Aceton umkristallisiert werden 5.8 g 3,3-Ethylendioxy-17α-(3-hydroxy-1-propinyl)-1α-methyl-5α-androstan-17β-ol vom Schmelzpunkt 232 °C erhalten.

D. 15 g 3,3-Ethylendioxy-17α-(3-hydroxy-1-propinyl)-1α-methyl-5α-androstan-17β-ol werden in 150 ml Tetrahydrofuran und 150 ml Methanol mit 15 ml 8prozentiger Schwefelsäure 1.5 Stunden bei Raumtemperatur gerührt. Die Reaktionslösung wird mit Diethylether verdünnt, mit Wasser neutral gewaschen, getrocknet und eingedampft. Es werden 12 g 17β-Hydroxy-17α-(3-hydroxy-1-propinyl)-1α-methyl-5α-androstan-3-on erhalten.

E. 1.0 g 17β-Hydroxy-17α-(3-hydroxy-1-propinyl)-1α-methyl-5α-androstan-3-on werden in 2 ml Pyridin mit 1 ml Propionsäureanhydrid 17 Stunden bei Raumtemperatur stehengelassen. Das Reaktionsgemisch wird im Eiswasser eingerührt, der ausgefallene Niederschlag wird abfiltriert, in Diethylether aufgenommen und die Lösung mit Natriumhydrogencarbonatlösung gewaschen. Nach dem Trocknen und Eindampfen wird der Rückstand chromatographiert. Es werden 950 mg 17β-Hydroxy-1α-methyl-17α-(3-propionyloxy-1-propinyl)-5α-androstan-3-on vom Schmelzpunkt 98 °C erhalten.

F. 750 mg 17β-Hydroxy-1α-methyl-17α-(3-propionyloxy-1-propinyl)-5α-androstan-3-on werden unter dem in Beispiel 12 A beschriebenen Bedingungen bis zur Aufnahme von zwei Äquivalenten Wasserstoff hydriert. Das Reaktionsprodukt wird an Kieselgel chromatographiert. Man erhält 673 mg 17β-Hydroxy-1α-methyl-17α-(3-propionyloxypropyl)-5α-androstan-3-on als Öl.

## Beispiel 5

A. 2.0 g 17β-Hydroxy-17α-(3-hydroxy-1-propinyl)-1α-methyl-5α-androstan-3-on werden in 60 ml Benzol und 40 ml Tetrahydrofuran mit 600 mg Lindlar-Katalysator bis zur Aufnahme von einem Äquivalent Wasserstoff hydriert. Es wird vom Katalysator abfiltriert und zur Trockne eingedampft. Der Rückstand wird mit Diisopropylether verrieben und abgesaugt. Es werden 1.75 g 17β-Hydroxy-17α-(3-hydroxy-1-propenyl)-1α-methyl-5α-androstan-3-on vom Schmelzpunkt 184 °C erhalten.

B. 800 mg 17β-Hydroxy-17α-(3-hydroxy-1-propenyl)-1α-methyl-5α-androstan-3-on werden in 1.6 ml Pyridin und 0.8 ml Propionsäure anhydrid, wie im Beispiel 10 E beschrieben, umgesetzt und aufgearbeitet. Nach der Chromatographie an Kieselgel werden 730 mg 17β-Hydroxy-1α-methyl-17α-(3-propionyloxy-1-propinyl)-5α-androstan-3-on vom Schmelzpunkt 87 °C erhalten.

C. 7.5 g 3,3-Ethylendioxy-17α-(3-hydroxy-1-propinyl)-1α-methyl-5α-androstan-17β-ol werden in 120 ml 2-Propanol und 120 ml Tetrahydrofuran mit 6 g Raney-Nickel-Katalysator bis zur Aufnahme von zwei Äquivalenten Wasserstoff hydriert. Danach wird vom Katalysator abfiltriert und im Vakuum zur Trockne eingedampft. Der Rückstand wird an Kieselgel chromatographiert. Nach dem Umkristallisieren aus Diisopropylether-Aceton werden 3.5 g 3,3-Ethylendioxy-17α-(3-hydroxypropyl)-1α-methyl-5α-androstan-17β-ol vom Schmelzpunkt 192 °C erhalten.

D. 3.3 g 3,3-Ethylendioxy-17α-(3-hydroxypropyl)-1α-methyl-5α-androstan-17β-ol werden in 66 ml Methanol mit 3.3 ml 8prozentiger Schwefelsäure eine Stunde bei Raumtemperatur gerührt. Die Reaktionslösung wird mit Diethylether verdünnt, mit Wasser gewaschen, getrocknet und eingedampft. Der Rückstand wird aus Diisopropylether-Aceton umkristallisiert. Ausbeute : 2.3 g 17β-Hydroxy-17α-(3-hydroxypropyl)-1α-methyl-5α-androstan-3-on vom Schmelzpunkt 163 °C.

E. 500 mg 17β-Hydroxy-17α-(3-hydroxypropyl)-1α-methyl-5α-androstan-3-on werden in 2 ml Pyridin und 1 ml Propionsäureanhydrid 17 Stunden bei Raumtemperatur stehengelassen. Es wird wie im Beispiel 10 E beschrieben aufgearbeitet. Nach Der Chromatographie an Kieselgel werden 460 mg 17β-Hydroxy-1α-methyl-17α-(3-propionyloxypropyl)-5α-androstan-3-on als Öl erhalten.

## Beispiel 6

1.0 g 17β-Hydroxy-17α-(3-hydroxypropyl)-1α-methyl-5α-androstan-3-on werden in einem Gemisch aus 4 ml Pyridin und 1 ml Buttersäureanhydrid 17 Stunden bei Raumtemperatur stehengelassen. Es wird wie im Beispiel 10 E beschrieben aufgearbeitet. Nach der Chromatographie an Kieselgel werden 860 mg 17α-(3-Butyryloxypropyl)-17β-hydroxy-1α-methyl-5α-androstan-3-on als Öl erhalten. $[\alpha]_D^{23} = +3.5°$ (C = 0.5 in Chloroform).

## Beispiel 7

800 mg 17β-Hydroxy-17α-(3-hydroxypropyl)-1α-methyl-5α-androstan-3-on werden in 1.6 ml Pyridin und 0.8 ml Capronsäureanhydrid 24 Stunden bei Raumtemperatur stehengelassen. Die Reaktionslösung wird in Eiswasser eingerührt, dann mit Diethylether extrahiert und die Etherphase mit Natriumhydrogencarbonatlösung und Wasser gewaschen. Nach dem Trocknen und Eindampfen wird der Rückstand an Kieselgel chromatographiert. Es werden 950 mg 17α-(3-Hexanoyloxypropyl)-17β-hydroxy-1α-methyl-5α-androstan-3-on als Öl erhalten. $[\alpha]_D^{23} = +3.8°$ (C = 0.5 in Chloroform).

## Beispiel 8

A. 10 g 3,3-Ethylendioxy-1α-methyl-5α-androstan-17-on werden in 100 ml absolutem Tetrahydrofuran gelöst. Anschließend werden 20 g Zink-Kupfer-Paar zugegeben und nach Zugabe von etwas Jod werden innerhalb 30 Minuten 40 ml Bromessigsäureethylester zugetropft. Nach Beendigung der zum Teil heftigen Umsetzung wird 30 Minuten bei Raumtemperatur nachgerührt. Anschließend wird gesättigte Ammoniumchloridlösung zugetropft, die Reaktionslösung mit Diethylether verdünnt und mit Wasser gewaschen. Nach dem Trocknen und Eindampfen wird der Rückstand an Kieselgel chromatographiert. Es werden 7.9 g (3,3-Ethylendioxy-17β-hydroxy-1α-methyl-5α-androstan-17α-yl)-essigsäureethylester als Öl erhalten.

B. 7.5 g (3,3-Ethylendioxy-17β-hydroxy-1α-methyl-5α-androstan-17α-yl)-essigsäureethylester werden in 225 ml absolutem Tetrahydrofuran unter Eiskühlung portionsweise mit 2.5 g Lithiumalanat versetzt. Es wird 1.5 Stunden unter Kühlung nachgerührt und dann werden nacheinander 2.5 ml Wasser, 2.5 ml 15prozentige Natronlauge und 7.5 ml Wasser zur Reaktionslösung gegeben. Anschließend wird vom ausgefallenen Niederschlag abgesaugt, mit Tetrahydrofuran nachgewaschen und im Vakuum zur Trockne eingedampft. Der Rückstand wird an Kieselgel chromatographiert und es werden 4.2 g 3,3-Ethylendioxy-17α-(2-hydroxyethyl)-1α-methyl-5α-androstan-17β-ol erhalten. Eine aus Aceton-Dichlormethan umkristallisierte Probe schmilzt bei 229 °C.

C. 4.0 g 3,3-Ethylendioxy-17α-(2-hydroxyethyl)-1α-methyl-5α-androstan-17β-ol werden in 80 ml Methanol und 20 ml Tetrahydrofuran mit 4 ml 8prozentiger Schwefelsäure 2 Stunden bei Raumtemperatur gerührt. Das Reaktionsgemisch wird mit Diethylether verdünnt, mit Wasser gewaschen, getrocknet und eingedampft. Als Rückstand werden 3.5 g 17β-Hydroxy-17α-(2-hydroxyethyl)-1α-methyl-5α-androstan-3-on vom Schmelzpunkt 192 °C erhalten.

D. 1.2 g 17β-Hydroxy-17α-(2-hydroxyethyl)-1α-methyl-5α-androstan-3-on werden in 4.8 ml Pyridin und 1.2 ml Propionsäureanhydrid 6 Stunden bei Raumtemperatur gerührt. Es wird wie in Beispiel 10 E beschrieben aufgearbeitet. Nach der Chromatographie an Kieselgel werden 1.26 g 17β-Hydroxy-1α-methyl-17α-(2-propionyloxyethyl)-5α-androstan-3-on vom Schmelzpunkt 124 °C erhalten.

## Beispiel 9

A. 1.5 g Lithium werden in 50 ml absolutem Tetrahydrofuran unter Argon mit 10 ml 1-Chlor-4-(1-ethoxyethoxy)-butan versetzt. Unter Eiskühlung werden 5.0 g 3,3-Ethylendioxy-1α-methyl-5α-androstan-17-on zugegeben und anschließend wird 3 Stunden bei Raumtemperatur nachgerührt. Die Reaktionslösung wird vom unumgesetzten Lithium abgegossen und in Eiswasser eingerührt. Die ausgefallene Substanz wird mit Diethylether extrahiert. Nach dem Trocknen und Eindampfen wird der Rückstand an Kieselgel chromatographiert. Es werden 2.4 g 17α-[4-(1-Ethoxyethoxy)-butyl]-3,3-ethylendioxy-1α-methyl- 5α-androstan-17β-ol als Öl erhalten.

B. 2.4 g 17α-[4-(1-Ethoxyethoxy)-butyl]-3,3-ethylendioxy-1α-methyl-5α-androstan-17β-ol werden in 24 ml Methanol mit 2.4 ml 8prozentiger Schwefelsäure eine Stunde bei Raumtemperatur gerührt. Es wird mit Diethylether verdünnt, mit Wasser gewaschen, getrocknet und eingedampft. Der Rückstand wird an Keiselgel chromatographiert. Aus Diisopropylether umkristallisiert, werden 1.3 g 17β-Hydroxy-17α-(4-hydroxybutyl)-1α-methyl-5α-androstan-3-on vom Schmelzpunkt 131 °C erhalten.

C. 1.1 g 17β-Hydroxy-17α-(4-hydroxybutyl)-1α-methyl-5α-androstan-3-on werden in 2.2 ml Pyridin und 1.1 ml Propionsäureanhydrid 4.5 Stunden bei Raumtemperatur stehengelassen. Es wird wie im Beispiel 10 E beschrieben aufgearbeitet. Nach der Chromatographie an Kieselgel werden 1.2 g 17β-Hydroxy-1α-methyl-17α-(4-propionyloxybutyl)-5α-androstan-3-on als Öl erhalten. $[\alpha]_D^{23} = +1.1°$ (C = 0.5 in Chloroform).

## Beispiel 10

700 mg 17β-Hydroxy-17α-(3-hydroxypropyl)-1α-methyl-5α-androstan-3-on werden in 2.8 ml Pyridin und 0.7 ml Acetanhydrid 16 Stunden beim Raumtemperatur stehengelassen. Es wird wie im Beispiel 10 E beschrieben aufgearbeitet und chromatographiert. Das Rohprodukt wird aus Diisopropylether umkristallisiert. Ausbeute 510 mg 17α-(3-Acetoxypropyl)-17β-hydroxy-1α-methyl-5α-androstan-3-on vom Schmelzpunkt 64 °C.

## Beispiel 11

A. Eine Lösung von 3.0 g 3.3-Ethylendioxy-1α-methyl-5α-androstan-17-on in 30 ml absolutem Tetrahydrofuran wird im Eisbad unter einem Argonstrom mit 9.0 g Kaliumethylat versetzt. Anschließend werden 4.5 ml 3-Butin-1-ol, in 9 ml absolutem Tetrahydrofuran gelöst, zugetropft und 2.5 Stunden bei Raumtemperatur gerührt. Das Reaktionsgemisch wird mit Dichlormethan verdünnt, die Lösung mit verdünnter Schwefelsäure und Wasser gewaschen, getrocknet und eingedampft. Der Rückstand wird an Kieselgel chromatographiert. Es werden 3.1 g 3.3-Ethylendioxy-17α-(4-hydroxy-1-butinyl)-1α-methyl-5α-androsten-17β-ol als Öl erhalten.

B. 640 mg 3.3-Ethylendioxy-17α-(4-hydroxy-1-butinyl)-1α-methyl-5α-androstan-17β-ol werden in einem Gemisch aus 2.7 ml Pyridin und 0.64 ml Propionsäureanhydrid 17 Stunden bei Raumtemperatur stehengelassen. Es wird wie im Beispiel 10 E beschrieben aufgearbeitet. Nach Chromatographie an

Kieselgel werden 540 mg 3.3-Ethylendioxy-1α-methyl-17α-(4-propionyloxy-1-butinyl)-5α-androstan-17β-ol als Öl erhalten.

C. 530 mg 3.3-Ethylendioxy-1α-methyl-17α-(4-propionyloxy-1-butinyl)-5α-androstan-17β-ol werden in 5.3 ml Methanol mit 0.53 ml 8prozentiger Schwefelsäure 45 Minuten bei Raumtemperatur gerührt. Es wird wie im Beispiel 10 D beschrieben, aufgearbeitet. Der Rückstand wird an Kieselgel chromatographiert. Man erhält 420 mg 17β-Hydroxy-1α-methyl-17α-(4-propionyloxy-1-butinyl)-5α-androstan-3-on vom Schmelzpunkt 120.5 °C.

D. 400 mg 17β-Hydroxy-1α-methyl-17α-(4-propionyloxy-1-butinyl)-androstan-3-on werden unter den im Beispiel 12 A beschriebenen Bedingungen bis zur Aufnahme von zwei Äquivalenten Wasserstoff hydriert. Das Rohprodukt wird an Kieselgel chromatographiert. Es werden 265 mg 17β-Hydroxy-1α-methyl-17α-(4-propionyloxy-butyl)-5α-androstan-3-on als Öl erhalten.

Beispiel 12

A. 800 mg 3.3-Ethylendioxy-17α-(4-hydroxy-1-butinyl)-1α-methyl-5α-androstan-17β-ol werden in 24 ml Toluol und 16 ml Tetrahydrofuran mit 240 mg Lindlar-Katalysator, wie im Beispiel 11A beschrieben, hydriert. Es werden 800 mg 3.3-Ethylendioxy-17α-(4-hydroxy-1-butenyl)-1α-methyl-5α-androstan-17β-ol erhalten.

B. Eine Lösung von 800 mg 3,3-Ethylendioxy-17α-(4-hydroxy-1-butenyl)-1α-methyl-5α-androstan-17β-ol in 8 ml Methanol wird mit 0.8 ml 8prozentiger Schwefelsäure versetzt und 2 Stunden bei Raumtemperatur gerührt. Das Reaktionsgemisch wird wie im Beispiel 10 D beschrieben aufgearbeitet und der Rückstand an Kieselgel chromatographiert. Man erhält 450 mg 17β-Hydroxy-17α-(4-hydroxy-1-butenyl)1α-methyl-5α-androstan-3-on.

Beispiel 13

A. Eine Lösung von 3.0 g 3,3-Ethylendioxy-1α-methyl-5α-androstan-17-on in 30 ml absolutem Tetrahydrofuran wird im Eisbad unter Argon mit 9.0 g Kaliumethylat versetzt. Anschließend werden 4.5 ml 4-Pentin-1-ol, in 9 ml absolutem Tetrahydrofuran gelöst, zugetropft. Das Reaktionsgemisch wird 4 Stunden bei Raumtemperatur gerührt und anschließend wie im Beispiel 11 A beschrieben aufgearbeitet. Der Rückstand wird an Kieselgel chromatographiert. Man erhält 2.4 g 3,3-Ethylendioxy-17α-(5-hydroxy-1-pentinyl)-1α-methyl-5α-androstan-17β-ol.

B. 2.3 g 3,3-Ethylendioxy-17α-(5-hydroxy-1-pentinyl)-1α-methyl-5α-androstan-17β-ol werden in einem Gemisch aus 24 ml Methanol und 24 ml Tetrahydrofuran mit 240 mg 10prozentigem Palladium auf Calciumcarbonat wie im Beispiel 12 A beschrieben hydriert. Das Rohprodukt wird an Kieselgel chromatographiert. Ausbeute : 2.2 g 3.3-Ethylendioxy-17α-(5-hydroxypentyl)-1α-methyl-5α-androstan-17β-ol.

C. Eine Lösung von 2.2 g 3,3-Ethylendioxy-17α-(5-hydroxypentyl)-1α-methyl-5α-androstan-17β-ol in 6,6 ml Pyridin und 2.2 ml Propionsäureanhydrid wird 16 Stunden bei Raumtemperatur stehengelassen und wie im Beispiel 10 E beschrieben aufgearbeitet und 2.7 g 3,3-Ethylendioxy-1α-methyl-17α-(5-propionyloxy-pentyl)-5α-androstan-17β-ol.

D. Eine Lösung von 2.7 g 3,3-Ethylendioxy-1α-methyl-17α-(5-propionyloxy-pentyl)-5α-androstan-17β-ol in 27 ml Methanol wird mit 2.7 ml 8prozentiger Schwefelsäure versetzt und eine Stunde bei Raumtemperatur stehengelassen. Das Reaktionsgemisch wird wie im Beispiel 10 D beschrieben aufgearbeitet und der Rückstand an Kieselgel chromatographiert. Ausbeute : 590 mg 17β-Hydroxy-1α-methyl-17α-(5-propionyloxypentyl)-5α-androstan-3-on als Öl.

**Patentansprüche**

1. Androstan-Derivate der allgemeinen Formel 1

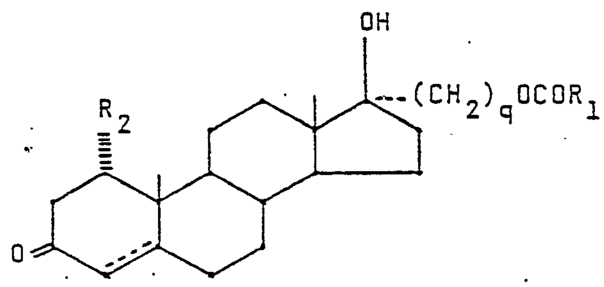

(I)

worin ..... eine Einfachbindung oder eine Doppelbindung $R_1$ einen Alkylrest mit 1 bis 8 Kohlenstoffatomen, q die Ziffern 3 oder 4 und $R_2$ ein Wasserstoffatom oder eine Methylgruppe darstellen.

2. 17α-(3-Acetoxypropyl)-17β-hydroxy-1α-methyl-4-androsten-3-on.
3. 17β-Hydroxy-1α-methyl-17α-(3-propionyloxypropyl)-4-androsten-3-on.
4. 17α-(3-Butyryloxypropyl)-17β-hydroxy-1α-methyl-4-androsten-3-on.
5. 17α-(3-Acetoxypropyl)-17β-hydroxy-5α-androstan-3-on.
6. 17β-Hydroxy-17α-(3-propionyloxypropyl)-5α-androstan-3-on.
7. 17α-(3-Butyryloxypropyl)-17β-hydroxy-5α-androstan-3-on.
8. 17β-Hydroxy-1α-methyl-17α-(3-propionyloxypropyl)-5α-androstan-3-on.
9. 17α-(3-Butyryloxypropyl)-17β-hydroxy-1α-methyl-5α-androstan-3-on.
10. 17α-(3-Hexanoyloxypropyl)-17β-hydroxy-1α-methyl-5α-androstan-3-on.
11. 17β-Hydroxy-1α-methyl-17α-(2-propionyloxyethyl)-5α-androstan-3-on.
12. 17β-Hydroxy-1α-methyl-17α-(4-propionyloxybutyl)-5α-androstan-3-on.
13. 17α-(3-Acetoxypropyl)-17β-hydroxy-4-androsten-3-on.
14. 17β-Hydroxy-17α-(3-propionyloxypropyl)-4-androsten-3-on.
15. 17α-(3-Butyryloxypropyl)-17β-hydroxy-4-androsten-3-on.
16. 17β-Hydroxy-17α-(3-trimethylacetoxypropyl)-4-androsten-3-on.
17. 17β-Hydroxy-18-methyl-17α-(3-propionyloxypropyl)-4-androsten-3-on.
18. 17β-Hydroxy-17α-(4-propionyloxybutyl)-4-androsten-3-on.
19. 17α-(3-Acetoxypropyl)-17β-hydroxy-1α-methyl-5α-androstan-3-on.
20. 17α-(4-Butyryloxybutyl)-17β-hydroxy-1α-methyl-5α-androstan-3-on.
21. 17β-Hydroxy-17α-(5-propionyloxypentyl)-1α-methyl-5α-androstan-3-on.

22. Pharmazeutische Präparate gekennzeichnet durch einen Gehalt von ein oder zwei Androstan-Derivaten gemäß Anspruch 1 bis 21.

23. Verfahren zur Herstellung von Androstan-Derivaten der allgemeinen Formel I gemäß Patentanspruch 1, dadurch gekennzeichnet, daß man in an sich bekannter Weise

a) ein Androstan-Derivat der allgemeinen Formel II

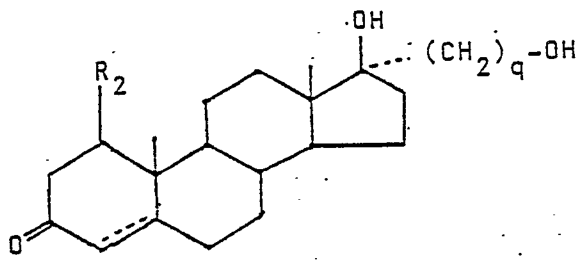

(II)

worin ....., $R_2$ und q die obengenannte Bedeutung besitzen, mit einer Säure der allgemeinen Formel III

$R_1COOH$ (III),

worin $R_1$ die obengenannte Bedeutung besitzt, oder einem reaktionsfähigen Derivat derselben, verestert oder

b) aus einem Androstan-Derivat der allgemeinen Formel IV

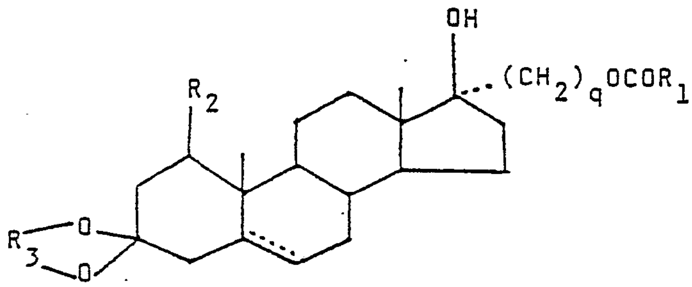

(IV)

worin ....., $R_1$, $R_2$ und q die obengenannte Bedeutung besitzen, und $R_3$ eine geradkettige oder verzweigte Alkylengruppe mit 2 bis 6 Kohlenstoffatomen darstellt, die Ketalgruppe hydrolytisch abspaltet, oder

c) ein Androstan-Derivat der allgemeinen Formel V

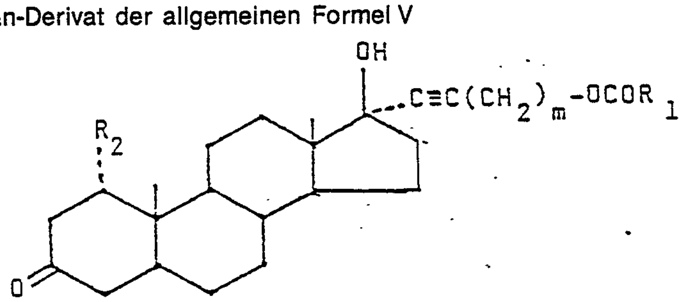

(V)

worin $R_1$ und $R_2$ die obengenannte Bedeutung besitzt und m die Ziffer q — 2 darstellt, zur Herstellung gesättigter 1α, 2α-Methylensteroide der allgemeinen Formel I hydriert.

## Claims

1. Androstane derivatives of the general formula I

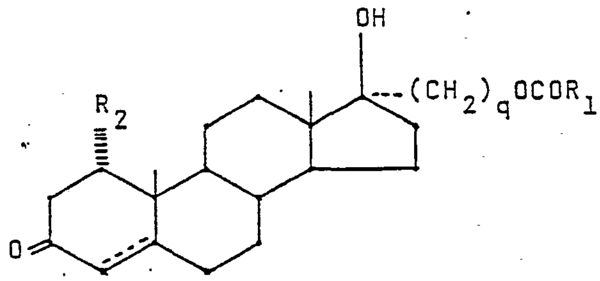

(I)

wherein ..... is a single or double bond, $R_1$ is an alkyl group of 1 to 8 carbon atoms, q is the integer 3 or 4 and $R_2$ is a hydrogen atom or a methyl group.

2. 17α-(3-Acetoxypropyl)-17β-hydroxy-1α-methyl-4-androsten-3-one.
3. 17β-Hydroxy-1-methyl-17α-(3-propionyloxypropyl)-4-androsten-3-one.
4. 17α-(3-Butyryloxypropyl)-17β-hydroxy-1α-methyl-4-androsten-3-one.
5. 17α-(3-Acetoxypropyl)-17β-hydroxy-5α-androstan-3-one.
6. 17β-Hydroxy-17α-(3-propionyloxypropyl)-5α-androstan-3-one.
7. 17α-(3-Butyryloxypropyl)-17β-hydroxy-5α-androstan-3-one.
8. 17β-Hydroxy-1α-methyl-17α-(3-propionyloxypropyl)-5α-androstan-3-one.
9. 17α-(3-Butyryloxypropyl)-17β-hydroxy-1α-methyl-5α-androstan-3-one.
10. 17α-(3-Hexanoyloxypropyl)-17β-hydroxy-1α-methyl-5α-androstan-3-one.
11. 17β-Hydroxy-1α-methyl-17α-(2-propionyloxyethyl)-5α-androstan-3-one.
12. 17β-Hydroxy-1α-methyl-17α-(4-propionyloxybutyl)-5α-androstan-3-one.
13. 17α-(3-Acetoxypropyl)-17β-hydroxy-4-androsten-3-one.
14. 17β-Hydroxy-17α-(3-propionyloxypropyl)-4-androsten-3-one.
15. 17α-(3-Butyryloxypropyl)-17β-hydroxy-4-androsten-3-one.
16. 17β-Hydroxy-17α-(3-trimethylacetoxypropyl)-4-androsten-3-one.
17. 17β-Hydroxy-18-methyl-17α-(3-propionyloxypropyl)-4-androsten-3-one.
18. 17β-Hydroxy-17α-(4-propionyloxybutyl)-4-androsten-3-one.
19. 17α-(3-Acetoxypropyl)-17β-hydroxy-1α-methyl-5α-androstan-3-one.
20. 17α-(3-Butyryloxypropyl)-17β-hydroxy-1α-methyl-5α-androstan-3-one.
21. 17β-Hydroxy-17α-(5-propionyloxypentyl)-1α-methyl-5α-androstan-3-one.
22. Pharmaceutical preparations characterised in that they comprise one or two androstane derivatives according to claims 1 to 21.
23. Process for the preparation of androstane derivatives of general formula I according to claim 1, characterised in that in known manner.

a) an androstane derivative of general formula II

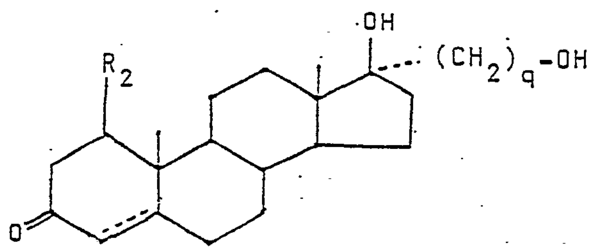

(II)

wherein ....., $R_2$ and q have the meanings given above, is esterified with an acid of general formula III

$R_1COOH$ (III)

wherein $R_1$ has the meaning given above, or a reactive derivative thereof, or

b) the ketal group is split off from an androstane derivative of general formula IV

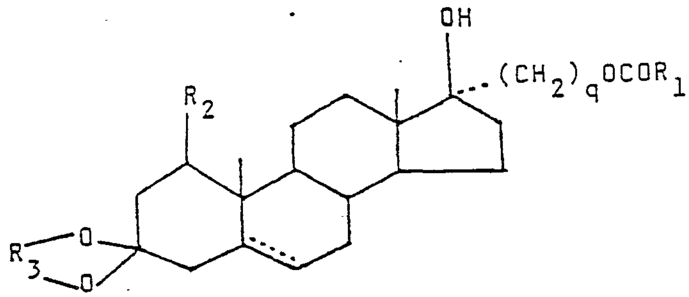

(IV)

wherein ..... $R_1$, $R_2$ and q have the meanings given above, and $R_3$ is a straight or branched chain alkylene group of 2 to 6 carbon atoms, or

c) for the preparation of a saturated 1α, 2α-methylene steroid of general formula I, an androstane derivative of general formula V

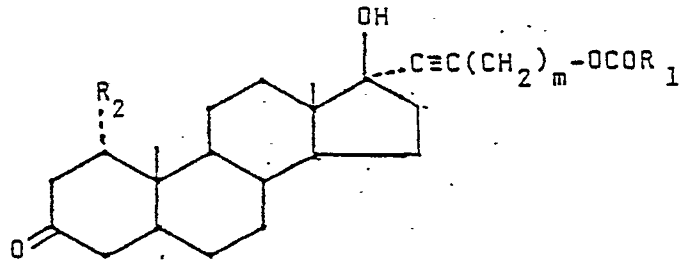

(V)

wherein $R_1$ and $R_2$ have the meanings given above and m is the integer q = 2, is hydrogenated.

**Revendications**

1. Dérivés de l'androstane de formule générale I ci-dessous :

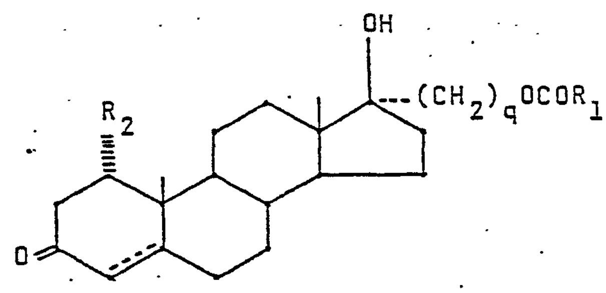

(I)

dans laquelle ..... représente une liaison simple ou double, $R_1$ un alkyle en $C_1$ à $C_8$, q le nombre 3 ou 4 et $R_2$ un atome d'hydrogène ou un groupe méthyle.

2. 17α-(3-acétoxypropyl)-17β-hydroxy-1α-méthyl-4-androstène-3-one.
3. 17β-hydroxy-1α-méthyl-17α-(3-propionyloxypropyl)-4-androstène-3-one.
4. 17α-(3-butyryloxypropyl)-17β-hydroxy-1α-méthyl-4-androstène-3-one.
5. 17α-(3-acétoxypropyl)-17β-hydroxy-5α-androstane-3-one.
6. 17β-hydroxy-17α-(3-propionyloxypropyl)-5α-androstane-3-one.
7. 17α-(butyryloxypropyl)-17β-hydroxy-5α-androstane-3-one.
8. 17β-hydroxy-1α-méthyl-17α-(3-propionyloxypropyl)-5α-androstane-3-one.
9. 17α-(3-butyryloxypropyl)-17β-hydroxy-1α-méthyl-5α-androstane-3-one.
10. 17α-(3-hexanoyloxypropyl)-17β-hydroxy-1α-méthyl-5α-androstane-3-one.
11. 17β-hydroxy-1α-méthyl-17α-(2-propionyloxyéthyl)-5α-androstane-3-one.
12. 17β-hydroxy-1α-méthyl-17α-(4-propionyloxybutyl)-5α-androstane-3-one.
13. 17α-(3-acétoxypropyl)-17β-hydroxy-4-androstène-3-one.
14. 17β-hydroxy-17α-(3-propionyloxypropyl)-4-androstène-3-one.
15. 17α-(3-butyryloxypropyl)-17β-hydroxy-4-androstène-3-one.
16. 17β-hydroxy-17α-(3-triméthylacétoxypropyl)-4-androstène-3-one.
17. 17β-hydroxy-18-méthyl-17α-(3-propionyloxypropyl)-4-androstène-3-one.
18. 17β-hydroxy-17α-(4-propionyloxybutyl)-4-androstène-3-one.

19. 17α-(3-acétoxypropyl)-17β-hydroxy-1α-méthyl-5α-androstane-3-one.
20. 17α-(4-butyryloxybutyl)-17β-hydroxy-1α-méthyl-5α-androstane-3-one.
21. 17β-hydroxy-17α(5-propionyloxypentyl)-1α-méthyl-5α-androstane-3-one.
22. Médicaments caractérisés en ce qu'ils contiennent un ou deux dérivés de l'androstane selon l'une quelconque des revendications 1 à 21 précédentes.
23. Procédé de préparation des dérivés de l'androstane de formule I selon la revendication 1, caractérisé en ce que de manière connue, ou bien :
a) On estérifie un dérivé d'androstane de formule générale II :

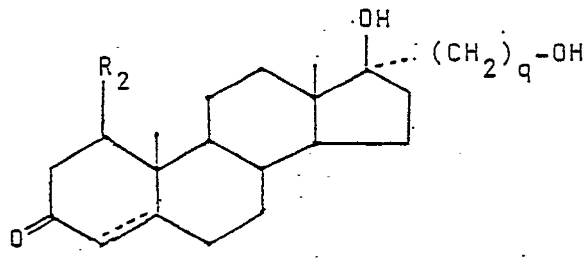

(II)

avec un acide de formule générale III :

$R_1COOH$ (III),

ou un dérivé réactif de cet acide, ou bien
b) On élimine par hydrolyse le groupe cétal d'un dérivé d'androstane de formule générale IV :

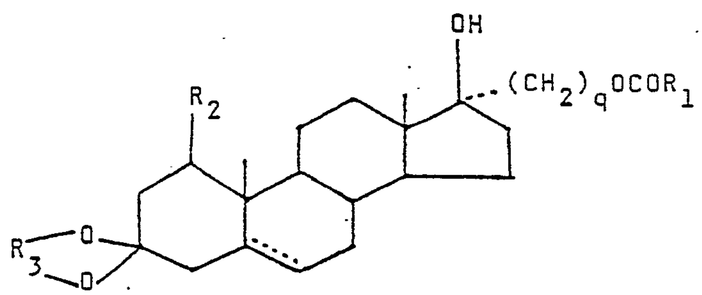

(IV)

$R_3$ représentant un alkylène à chaîne droite ou ramifiée en $C_2$ à $C_6$, ou encore
c) On hydrogène un dérivé d'androstane de formule générale V :

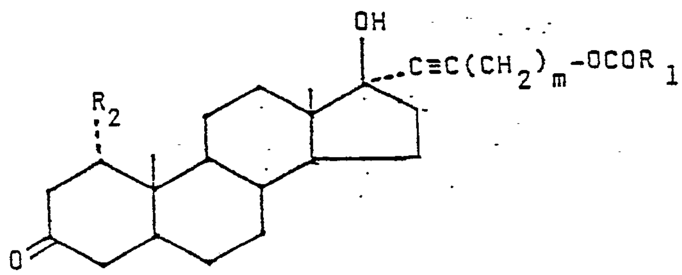

(V)

m étant le nombre q — 2, pour obtenir des 1α, 2α-méthylène-stéroïdes saturés de formule I.